# EUROPEAN PATENT APPLICATION

(11) **EP 3 563 695 A1**
(43) Date of publication of application: **06.11.2019**
(21) Application number: 17886920.2
(22) Date of filing: 21.09.2017
(51) Int. Cl.: A23L 27/10, A23L 2/00, A23L 2/02, A23L 19/00, A23L 27/00, C12P 21/06

(54) **PERICARP EXTRACT AND MANUFACTURING METHOD THEREFOR**

(30) Priority: 27.12.2016 JP 2016252277
(71) Applicant: Suntory Holdings Limited, Osaka-shi, Osaka 530-8203 (JP)
(72) Inventor: URAI, Soichiro, Kawasaki-shi Kanagawa 211-0067 (JP); NISHIBORI, Tomoyuki, Kawasaki-shi Kanagawa 211-0067 (JP); NAGAO, Koji, Kawasaki-shi Kanagawa 211-0067 (JP); HASHIMOTO, Takuya, Valencia 46749 (ES)
(74) Representative: Vossius & Partner Patentanwälte Rechtsanwälte mbB
(86) International application number: PCT/JP2017/034001
(87) International publication number: WO 2018/123159

(57) **Abstract**

An object of the present invention is to provide a technique for producing a pericarp extract excellent in flavor from citrus pericarp.

According to the present invention, an excellent pericarp extract can be obtained by subjecting citrus pericarp with a size of not more than 5 cm to enzymatic treatment with a hemicellulase and/or a protease, followed by solid-liquid separation.

## Description

### TECHNICAL FIELD

The present invention relates to a pericarp extract (peel extract) and a method for producing the same. This invention also relates to a beverage and food comprising a citrus pericarp extract and a method for producing the same.

### BACKGROUND ART

It has heretofore been common to extract a taste or aroma component from a citrus fruit and to add the obtained extract and other additives to a beverage, food or the like. For example, PTL 1 proposes efficient production of a processed pericarp product through enzymatically treating citrus pericarp with a pectinase to break down the fiber (pectin) in the pericarp. PTL 2 proposes that fruit juice with enhanced taste is extracted from a fruit and added to a beverage or the like to thereby add a natural taste to the beverage or the like and enhance the flavor of the beverage or the like.

For example, it is known that an aromatic essence composed of an aroma component concentrate is obtained from a citrus fruit juice used as a source material (NPL 1: J. Agric. Food Chem. 1990, 38, 2181). Further, besides the extraction of an aroma component from a citrus fruit juice using an organic solvent, it is known to extract an aroma component using a countercurrent contact apparatus (*e.g*., spinning cone column (SCC)) with the aim of suppressing change in flavor caused by heating while efficiently collecting a low-boiling point fraction (PTLs 3 to 5).

### CITATION LIST

### PATENT LITERATURES

PTL 1: Japanese Unexamined Patent Application Publication No. JP 2016-093111
PTL 2: Japanese Unexamined Patent Application Publication No. JP 2016-154520
PTL 3: International Patent Publication No. WO 90/02493
PTL 4: Japanese Examined Patent Application Publication No. JP H07-22646
PTL 5: Japanese Unexamined Patent Application Publication No. JP 2001-152180

### NON PATENT LITERATURE

NPL 1: J. Agric. Food Chem. 1990, 38,2181

### SUMMARY OF INVENTION

### TECHNICAL PROBLEM

Citrus pericarps are rich in bitterness components such as limonene, flavonoids such as hesperidin, and aroma components such as dietary fiber serving as the source of flavor. However, because of containing lots of relatively hard fiber mainly composed of pectin, citrus pericarps are difficult to utilize and thus have been discarded in many cases.

When fruit juice with strong acidic taste is added to a beverage for the purpose of taste enhancement, the beverage deteriorates in overall flavor balance due to the acidic taste derived from fruit juice; so, there is a limit to the amount added of such fruit juice. Further, since such fruit juice with strong acidic taste contains lots of organic acids, addition of such fruit juice results in a decrease in the pH of a beverage, leading to deterioration of an aroma component and beverage quality, and adversely affecting beverage stability; so, there is also a limit to the type of a beverage to which to add such fruit juice.

In view of these circumstances, an object of the present invention is to provide a technique for producing an extract excellent in flavor not from fruit pulp or juice but from citrus pericarp. In particular, this invention has as its object to provide a technique for producing an extract from citrus pericarp used as a source material.

### SOLUTION TO PROBLEM

The present inventors have made intensive studies to achieve the aforementioned object, and as a result found that an excellent pericarp extract can be obtained by subjecting citrus pericarp to enzymatic treatment with a hemicellulase and/or a protease followed by solid-liquid separation. Thus, the inventors have completed the present invention.

The present invention includes, but is not limited to, the following.
(1) A method for producing a pericarp extract, the method comprising the steps of:
   subjecting citrus pericarp made into a size of not more than 5 cm to enzymatic treatment with a hemicellulase and/or a protease to give an enzymatically treated slurry; and
   subjecting the enzymatically treated slurry to solid-liquid separation to give a pericarp extract.
(2) The method as set forth in (1), wherein the solid-liquid separation is performed using a filter press and/or centrifugation.
(3) The method as set forth in (1) or (2), wherein the citrus pericarp comprises any one or two or more of lemon pericarp, grapefruit pericarp, pink grapefruit pericarp, and orange pericarp.
(4) The method as set forth in any of (1) to (3), wherein the citrus pericarp comprises lemon pericarp.
(5) The method as set forth in any of (1) to (4), wherein 90% of particles present in the enzymatically treated pericarp slurry have a particle size of not more than 500 µm.
(6) A pericarp extract produced by the method as set forth in any of (1) to (5).
(7) The pericarp extract as set forth in (6), wherein the pericarp extract has a weight ratio of (citric acid and malic acid)/amino acids of not more than 10.
(8) A method for producing a beverage or food, the method comprising adding a pericarp extract produced by the method as set forth in any of (1) to (5) to a beverage or food.
(9) The method as set forth in (8), wherein the pericarp extract is added to the beverage so as to give a weight ratio of (citric acid and malic acid)/amino acids of not more than 14.
(10) A method for enhancing the taste of a beverage or food, the method comprising:
   producing a pericarp extract by the method as set forth in any of (1) to (5), and adding the pericarp extract to the beverage or food.

### ADVANTAGEOUS EFFECTS OF INVENTION

According to the present invention, a pericarp extract can be efficiently obtained from a citrus pericarp used as a source material. Since the pericarp extract of this invention is rich in amino acids, addition of this pericarp extract to a beverage or food enhances the taste of the beverage or food.

Further, according to the present invention, since an excellent extract can be obtained from a citrus pericarp used as a source material, it becomes possible to effectively utilize pericarps as a resource. Therefore, this invention is also useful particularly from the viewpoint of waste reduction.

### BRIEF DESCRIPTION OF DRAWINGS

[FIG. 1] FIG. 1 shows a photograph showing the appearances of the pericarp extracts obtained in Experiment 2-1 (starting from the left: hemicellulase + pectinase-treated slurry, hemicellulase alone-treated slurry, pectinase alone-treated slurry, and cellulase alone-treated slurry).
[FIG. 2] FIG. 2 shows a graph showing the particle size distributions of the pericarp slurries obtained in Experiment 3-2 (left: w/ grinding and hemicellulase treatment; right: w/ millstone grinding alone).
[FIG. 3] FIG. 3 shows photos showing the appearances of the pericarp slurries obtained in Experiment 3-3 (left: after enzymatic treatment in Experiment A; right: after enzymatic treatment in Experiment B).
[FIG. 4] FIG. 4 shows a graph showing the amino acid concentrations in the pericarp extracts obtained in Experiment 3-5.

### DESCRIPTION OF EMBODIMENTS

### Pericarp extract

The present invention relates to a pericarp extract obtained from citrus pericarp. The pericarp extract of this invention can be produced by a method comprising the steps of subjecting citrus pericarp with a size of not more than 5 cm to enzymatic treatment with the particular enzyme(s), and performing solid-liquid separation to give a pericarp extract.

In the present invention, citrus pericarp is used as a source material. Citrus is a group of plants belonging to the genera *Citrus, Fortunella, Poncirus,* etc. in the family *Rutaceae,* Subfamily *Aurantioideae,* tribe *Citreae.* Preferred examples thereof are *Citrus unshiu, Citrus depressa, Citrus reticulata varpoonensis, Citrus reticulata, Citrus tankan, Citrus junos, Citrus sudachi,* kumquat (*Fortunella*), *Citrus maxima, Citrus natsudaidai, Citrus aurantium,* lime (*Citrus aurantiifolia*), lemon (*Citrus limon*), *Citrus sphaerocarpa,* orange (*Citrus sinensis*), grapefruit (C*itrus* × *paradisi*), and the like, with lemon, orange, and grapefruit being particularly preferred. The citrus pericarp used in this invention can be any of its three distinct layers: epicarp (flavedo) which is the outermost layer, endocarp (albedo) which is the innermost layer, and mesocarp which is located between the foregoing layers. The citrus pericarp used as a source material can be obtained by removing fruit pulp and seeds from a citrus fruit, but may contain fruit pulp and/or juice. However, since the pericarp is a fruit part that is richest in protein, it is desirable that the pericarp be treated to be free of fruit pulp and juice as much as possible.

In the present invention, the citrus pericarp is first physically treated, but the method and degree of the physical treatment are not particularly limited. By subjecting the citrus pericarp to physical treatment followed by enzymatic treatment, it becomes possible to allow the enzyme(s) to act on the pericarp more efficiently. The citrus pericarp can be made into the form of dice, chop, pulp, powder or the like by physical treatment such as cutting, fracturing, grinding, or crushing. In a preferred mode, the citrus pericarp can be physically treated using a mill, millstone, or other milling apparatus. The size of the physically treated pericarp is not particularly limited as long as it is treated to have a size of not more than 5 cm. The pericarp is preferably crushed into a size of not more than 4 cm, more preferably not more than 3 cm, still more preferably not more than 1 cm, or may be physically treated to have a size of even not more than 5 mm or 3 mm. There is no lower limit on the size of the physically treated pericarp, but the physically treated pericarp preferably has a size of not less than 0.5 mm, more preferably not less than 0.7 mm, still more preferably not less than 1.0 mm. In this invention, it is preferred that the size of the physically treated pericarp fall within the range defined above, from the viewpoints of improvement of handleability and efficient progress of the subsequent enzymatic treatment.

In the present invention, citrus pericarp is enzymatically treated with a hemicellulase and/or a protease. Enzymatically treating citrus pericarp is particularly useful for production purpose, since this treatment makes it easier to elute an aroma component present in the pericarp and also causes the breakdown of citrus pericarp leading to an increase in the flowability of a pericarp slurry. The amount of enzyme used is not particularly limitedthis amount is not uniquely determined and varies with various treatment conditions, including the titer of the enzyme used, the type of the citrus pericarp used, and the degree of crushing. However, the enzyme is used in an amount ranging from 0.001 to 6 wt.%, more preferably from 0.01 to 5 wt.%, still more preferably from 0.1 to 4 wt.%, with respect to the weight of citrus pericarp.

The type of hemicellulase used in the present invention is not particularly limited and any such known enzyme can be used, as long as it is an enzyme capable of hydrolyzing hemicelluloses. Hemicellulases are enzymes capable of degrading hemicelluloses which are polysaccharides such as xylans, arabinoxylans, xyloglucans and glucomannans, and known examples of hemicellulases include xylanases and galactanases.

In the present invention, the "protease" refers to a peptide bond hydrolase, and is also called "proteinase". The protease used in this invention can be an exopeptidase which cleaves an amino acid residue(s) at the terminal ends of a peptide chain, or an endopeptidase which cleaves an amino acid residue(s) in the middle of a peptide chain, or can be a combination thereof. Also, the peptidase may be a plant-derived one, for example, a peptidase derived from papaya, pineapple, ginger, ficus, kiwi fruit, *Grifola frondosa,* rice malt, a fungus, or a bacterium such as *Bacillus subtilis* var. *natto.*

As long as a hemicellulase and/or a protease is used in the enzymatic treatment of the present invention, any other enzymes may also be used in combination, such as xylases, mannanases, cellulases, and pectinases. The amount of other enzymes combined is not particularly limited, and can be adjusted depending on the type of citrus pericarp used, and/or the like. For example, such other enzymes are used in an amount ranging from 0.001 to 6 wt.%, more preferably from 0.01 to 5 wt.%, still more preferably from 0.1 to 4 wt.%, with respect to the weight of citrus pericarp.

When a hemicellulase and a protease are used in combination in the enzymatic treatment of the present invention, the amino acid concentration in a pericarp extract is particularly enhanced. When these enzymes are used in combination, hemicellulose treatment may be performed followed by protease treatment, or the two treatments may be performed at the same time. Performing hemicellulase and protease treatments at the same time is advantageous, since doing so makes it possible to obtain an amino acid-rich pericarp extract in a relatively short time, and also to reduce the time of holding a pericarp slurry in a temperature zone suitable for the growth of microorganisms, thereby suppressing the growth of microorganisms.

In general, enzymatic treatment with a pectinase is, in some cases, done for the purpose of enhancing the flowability of a slurry of source material. However, the pectinase treatment is not necessarily required in the present invention, since the flowability of a slurry is fully enhanced by milling and hemicellulase treatment. Also, performing hemicellulase treatment offers additional advantages because of clarification of a supernatant and an increase in the amount of amino acids obtained.

The type of water used in a slurry containing citrus pericarp during enzymatic treatment is not particularly limited, and any type of water can be used as long as it does not adversely affect flavor. Examples thereof include tap water, ion exchanged water, soft water, distilled water, and degassed water obtained by degassing any of the aforementioned types of water. The concentration of an aqueous slurry containing citrus pericarp is not particularly limited, but is preferably in the range of 5 to 70%, more preferably 10 to 60%, still more preferably 15 to 55%, in consideration of flowability and treatment efficiency.

In the present invention, citrus pericarp used as a source material may be added in multiple additions with the aim of allowing an enzymatic reaction to proceed efficiently. The number of times of addition is not particularly limited, and for example, the pericarp as a source material may be added in 1 to 10 additions, in 1 to 5 additions, or in 2 to 4 additions.

Also, in order to allow an enzymatic reaction to proceed efficiently, it is preferred to perform enzymatic treatment under stirring. The type of stirrer used is not particularly limited, and for example, a vertical stirrer, a horizontal stirrer, a magnetic stirrer, a shaker, or the like can be used. Further, for the purpose of allowing an enzymatic reaction to proceed efficiently, the temperature of a reaction solution is preferably adjusted to be in the range of 15 to 70°C using a heater. The type of heater used is not particularly limited, and examples thereof include a steam heater, an electrothermal heater, a water jacket heater, and an electromagnetic heater. The treatment time is not particularly limited. For example, the treatment time is preferably in the range of 1 to 24 hours, more preferably 2 to 18 hours, or may also be set to 10 to 16 hours. The lower the reaction solution's temperature is, the longer the treatment time is, whereas the higher the reaction solution's temperature is, the shorter the treatment time is. Thus, the reaction temperature and time can be adjusted as appropriate. For example, when the enzymatic treatment is performed at 40°C, the enzymatic reaction can be completed in about 2 hours.

In the present invention, in order to terminate the enzymatic treatment, high temperatures can be applied to deactivate an enzyme. For example, the reaction can be controlled by heating a reaction solution to a temperature exceeding 80°C to induce enzyme deactivation. The time for deactivation is not particularly limited, and for example, deactivation treatment can be carried out for 30 seconds to 1 hour, preferably for 1 minute to 30 minutes, more preferably within 10 minutes. The conditions for deactivation vary with the heat resistance of an enzyme. For example, enzyme activity can be deactivated under the following conditions: at 80°C for 30 minutes; at 95°C for 5 minutes; or at 100°C for 1 minute. Enzyme deactivation can be carried out using a plate heater or a countercurrent contact apparatus. It is advantageous to use a countercurrent contact apparatus, because it becomes possible not only to induce enzyme deactivation but also to collect a desired aroma component in advance from a pericarp extract. Examples of such a countercurrent contact apparatus include spinning cone column (SCC).

In the present invention, solid-liquid separation can be performed using any known method without limitation. In a preferred mode, solid-liquid separation can be performed using a filter press, centrifugation, multistage filtration, or the like. By using a continuous centrifugal separator, large amounts of solid and liquid fractions can be separated in a short time.

### Amino acids

In the present invention, the "amino acids" refers to the principal amino acids constituting a protein, and specific examples thereof are arginine (Arg), lysine (Lys), histidine (His), phenylalanine (Phe), tyrosine (Tyr), leucine (Leu), isoleucine (Ile), methionine (Met), valine (Val), alanine (Ala), glycine (Gly), proline (Pro), glutamic acid (Glu), glutamine (Gln), serine (Ser), threonine (Thr), aspartic acid (Asp), asparagine (Asn), tryptophan (Trp), and cysteine (Cys). Unless otherwise specified, the amino acid content or concentration, as referred to in this invention, means the total content or concentration of the 20 different amino acids mentioned above. The amino acids present in a pericarp extract or the like can be quantified by HPLC or other means. In a preferred mode, the amino acid concentration in the pericarp extract of this invention is not less than 1500 ppm, preferably not less than 2000 ppm, more preferably not less than 2500 ppm, or may be not less than 3000 ppm. The upper limit of the amino acid concentration is not particularly limited, and can be, for example, not more than 10000 ppm, not more than 8000 ppm, or not more than 6000 ppm.

In the present invention, the "organic acids" refers to the principal organic acids that can be found in fruit juice, and specific examples thereof include phosphoric acid, citric acid, malic acid, chinic acid, formic acid, acetic acid, and propionic acid. Examples of the principal organic acids that can be found in a beverage include citric acid and malic acid. In a preferred mode, the pericarp extract of this invention has a weight ratio of (citric acid and malic acid)/amino acids of not more than 10, preferably not more than 8, more preferably not more than 6, still more preferably not more than 4. When the pericarp extract has such a weight ratio, it becomes easy to add a profound taste to a beverage or food without making the beverage or food taste prominently acidic.

The amount of citrus pericarp used to produce the citrus pericarp extract of the present invention is in the range of 1 to 90 mass%, preferably 20 to 60 mass%, with respect to the total amount of the citrus pericarp extract. The amount of water used is in the range of 1 to 90 mass%, preferably 20 to 60 mass%, with respect to the total amount of the pericarp extract. If the amount of citrus pericarp used falls below 20 mass% with respect to the total amount of the pericarp extract, the flavor of a beverage or food may become less intense, and also some disadvantages in cost and quality terms may occur because of the necessity for a concentration step. If the amount of water used falls below 20 mass% with respect to the total amount of the pericarp extract, a mixture of citrus pericarp and water may not have sufficient flowability for enzymatic treatment. Further, with the aim of improving reaction efficiency at the step of enzymatically treating citrus pericarp after mixed with water, a fracturing step may be further performed. In this invention, the proportion of citrus pericarp with respect to a processed citrus pericarp product is referred to as "slurry ratio". The higher the proportion of citrus pericarp, the higher the slurry ratio, whereas the lower the proportion of citrus pericarp, the lower the slurry ratio.

In one aspect, the present invention relates to a pericarp extract obtained in such a manner as described above. In this invention, a high-quality pericarp extract can be produced by a method comprising the steps of subjecting citrus pericarp with the particular size to enzymatic treatment with the particular enzyme(s), and performing solid-liquid separation to give a pericarp extract. The pericarp extract of this invention is typically in liquid form, but can be made into the form of, for example, paste, powder, or granule by being subjected to concentration, drying, granulation, or other treatment.

With regard to the pericarp extract according to the present invention, so-called product-by-product limitations may be used to define the invention. The reason for this is as follows. Since the quality of a pericarp extract is an indicator that relies on human senses, it is difficult to quantitatively define the whole scope of the pericarp extract only by its amino acid content as described below. Further, natural product-derived compositions like pericarp extracts contain not only amino acids but also a wide variety of chemical substances that affect flavor, and the flavor varies with the interaction between such chemical substances -- these facts are common technical knowledge available in the food industry. In particular, it is considered that various components present in an enzymatically treated product contribute to the quality of the pericarp extract according to this invention -- such an enzymatically treated product contains numerous types of components, including trace components below the limit of detection of the analyzer. However, with the scientific techniques available upon filing of the present application, it may well be considered impossible to completely analyze and identify which chemical substances among such numerical types of components present in an enzymatically treated product contribute to the effects of the present invention, because there are too many types of components present in an enzymatically treated product and also trace components below the limit of detection are not analyzable. Even if all of the components constituting an enzymatically treated product can be identified by, for example, making full use of analyzers with an extremely low limit of detection, the flavor of a pericarp extract is in many cases formed by mingling of multiple chemical substances; thus, no active component can be identified simply by determining the flavors of individual constituents. Therefore, with regard to the pericarp extract according to this invention, due to the existence of so-called "impractical circumstances", it should be acceptable to describe the invention using product-by-process limitations.

### Beverage

The pericarp extract of the present invention can be added to a beverage or food, and is particularly preferably added to a beverage. The amount of the pericarp extract added to a beverage or food can be selected, as appropriate, from for example the range of 0.1 to 5 w/v%. In a preferred mode, the pericarp extract is added to a beverage so as to give a weight ratio of (citric acid and malic acid)/amino acids of not more than 14. It is also preferable to add the pericarp extract to a beverage so as to give a weight ratio of (citric acid and malic acid)/amino acids of not more than 10 or not more than 6. The beverage can be an alcoholic beverage containing an alcohol, or a non-alcoholic beverage free of alcohol. It should be added that the alcohol as referred to in relation to beverages means ethyl alcohol (ethanol), unless otherwise specified.

The "alcoholic beverage" refers to a beverage containing an alcohol, and is produced by mixing an alcohol, as necessary, with water, fruit juice, a flavor, a sugar, a sweetener, an acidulant, and other ingredients. Preferred examples of the alcoholic beverage include, but are not limited to, spirits-based and liqueur-based beverages, such as shochu-based beverages, cocktails, fizz, and wine cooler. The type of an alcohol material is not particularly limited, and examples include distilled alcohols, spirits (e.g., rum, vodka, gin), liqueurs, whiskey, brandy, and shochu (e.g., continuous distilled shochu, single distilled shochu), with further examples being brewed beverages such as sake, wine, and beer. Such alcohol materials may each be used alone, or two or more thereof may be used in combination. It is preferable to select such an alcohol material that can retain its flavor. The alcohol concentration in an alcoholic beverage is not particularly limited, but is preferably not more than 30 v/v%, more preferably not more than 20 v/v%, still more preferably not more than 10 v/v%.

The beverage of the present invention may not necessarily contain fruit juice. When fruit juice is added, the amount of fruit juice added is preferably in the range of 0.01 to 30%, or may be not more than 20% or not more than 10%, in terms of straight fruit juice. With regard to the type of fruit juice, the fruit juice can be of any forms, including straight fruit juice used as freshly squeezed from a fruit, or concentrated fruit juice obtained by concentrating straight fruit juice. Further, cloudy fruit juice may be used, or use may be made of fruit juice from whole fruit, prepared by crushing the whole fruit including husk and simply removing particularly coarse solid matters like seeds, a fruit puree prepared by sieving a fruit, or fruit juice obtained by crushing or extracting a dried fruit pulp.

The type of fruit juice that can be used in the present invention is not particularly limited, and one or two or more types of fruit juice may be used. Preferred examples of fruit juice include, but are not limited to: juices from citrus fruits (*e.g.,* lemon (*Citrus limon*), grapefruit (*Citrus* × *paradisi*) (white, ruby), lime (*Citrus aurantiifolia*), orange (*Citrus sinensis*), *Citrus unshiu,* tangors, *Citrus natsudaidai, Citrus natsudaidai* Hayata, *Citrus hassaku, Citrus tamurana, Citrus depressa, Citrus sudachi, Citrus junos, Citrus sphaerocarpa, Citrus aurantium, Citrus iyo, Citrus reticulata* var *poonensis, Fortunella, Citrus sulcata* hort. ex. Ik. Takahashi, oroblanco, *Citrus maxima);* juices from drupes (*e.g.,* apricot (*Prunus armeniaca*), cherry (*Prunus avium*), Japanese apricot (*Prunus mume*), Japanese plum (*Prunus salicina*), peach (*Prunus persica*)); and juices from berries (*e.g.,* Muscat, Riesling, Delaware, Kyoho, Pione). When the beverage of this invention contains a lemon pericarp extract, citrus fruit juice is preferably added to the beverage, and fruit juice from lemon, grapefruit (white, ruby) or the like is particularly preferably added to the beverage.

The beverage of the present invention may contain a sugar including glucose. In the beverage of this invention, one or two or more types of natural or artificial sweeteners can be used. The type of sweetener used is not particularly limited. Examples of natural sweeteners include, but are not limited to, fructose, glucose, maltose, sucrose, high-fructose syrup, fructose-glucose syrup, glucose-fructose syrup, sugar alcohol, oligosaccharide, honey, sugarcane squeeze (brown sugar syrup), sugar (*e.g.,* saccharose, yellow soft sugar, brown sugar, Wasanbon), maple syrup, molasses, starch syrup, stevia powder, stevia extract, *Momordica grosvenori* powder, *Momordica grosvenori* extract, licorice (*Glycyrrhiza glabra*) powder, licorice extract, *Thaumatococcus daniellii* seed powder, and *Thaumatococcus daniellii* seed extract. Examples of artificial sweeteners include, but are not limited to, sucralose, acesulfame-K, neotame, aspartame, and saccharin. In the beverage of this invention, fructose and/or glucose is preferably added.

Examples of sugars that can be advantageously used in the present invention include, but are not limited to: monosaccharides such as ribose, xylose, arabinose, glucose, fructose, rhamnose, galactose, and 1,3-dihydroxyacetone; and disaccharides such as sucrose, lactose, and maltose. One of such sugars may be used alone, or a mixture of two or more thereof may be used.

Similarly to common beverages, the beverage of the present invention may have different additives added thereto to the extent that they do not impair the effects of this invention. Examples of different additives include, but are not limited to, acidulant, flavor, vitamin, colorant, antioxidant, emulsifier, preservative, seasoning, essence, pH adjustor, and quality stabilizer.

A thickener may also be used, and examples include, but are not limited to, pectin (*e.g.,* LM or HM pectin), sodium alginate, guar gum, tamarind seed gum, locust bean gum, xanthan gum, carrageenan, gellan gum, celluloses, and pregelatinized starches. Depending on the desired viscosity and the like, a suitable thickener can be selected as appropriate, or different thickeners may be used in combination. The amount of thickener used is not uniquely determined and varies with the type of thickener used and the like, but is typically in the range of 0.01 to 3 mass%, preferably 0.05 to 0.5 mass%.

The beverage of the present invention is advantageously provided as a packaged beverage. As referred to herein, the "packaged beverage" refers to a beverage that is packed in a package such as PET bottle, can, glass bottle or paper package and is drinkable without dilution. The packaged beverage is produced by packing a liquid preparation obtained in a preparation step into a package.

The package used to pack the beverage of the present invention can be any of commonly used packages, including metal packages such as aluminum and steel cans, resin packages such as PET bottle, glass bottle, and paper packages. In one mode, the package used to pack the beverage may be a clear package (e.g., PET bottle, glass bottle). Further, aseptic packaging at low temperatures may be adopted in this invention.

The packaged beverage of the present invention not only can be drunk directly from a package, but also can be consumed for drinking purpose by pouring the beverage packed in a bulk package such as bag-in-box or in a portion package into a different container. Also, a liquid concentrate of the inventive beverage can be diluted when consumed for drinking purpose.

In a preferred mode, the beverage of the present invention is a beverage that can retain its flavor perceived immediately after production even when it is stored for a long period of time at ordinary temperatures. For the purpose of long-term storage at ordinary temperatures, the beverage is heat sterilized during production. In a preferred mode of this invention, the beverage is heat sterilized, and as referred to herein, the "heat sterilization" can be exemplified by a process in which a beverage is sterilized at high temperatures for a short period and then packed in a sterilized storage package under aseptic conditions (UHT sterilization), and by a retort sterilization process in which a liquid preparation is packed in a storage package such as can and then the package is subjected to retort sterilization treatment. The conditions for heat sterilization can be selected, as appropriate, depending on the characteristics of a liquid preparation for beverage and the type of storage package used. The conditions for UHT sterilization are generally approximately at 90 to 150°C for 1 to 120 seconds, preferably approximately at 100 to 140°C for 1 to 90 seconds, and the conditions for retort sterilization are generally approximately at 110 to 130°C for 10 to 30 minutes, preferably approximately at 120 to 125°C for 10 to 20 minutes.

In a preferred mode, the packaged beverage of the present invention has a Brix value of 3 to 9. The concentration of soluble solids in solution can be evaluated by means of Brix values determined using a sugar content meter, refractometer, etc. The Brix is a value obtained by converting a refractive index measured at 20°C into a mass/mass percentage of sucrose in solution based on the conversion table published by ICUMSA (the International Commission for Uniform Methods of Sugar Analysis). The Brix is expressed in unit of "°Bx", "%" or "degree".

The beverage of the present invention may also be a low-solute beverage with a low concentration of soluble solids in solution -- thus, the beverage of this invention includes co-called low-calorie beverages labeled as "sugar-free", "saccharide-free", "low-calorie" or the like. The labelings as "sugar-free", "saccharide-free", "low-calorie" or the like are defined in the nutrition labeling standards as stipulated under the health promotion act. For example, the label as "sugar-free" is placed on beverages whose content of a sugar (which is a monosaccharide or disaccharide, and is not a sugar alcohol) is less than 0.5 g per 100 g of beverage. The label as "saccharide-free" is placed on beverages with a saccharide concentration of less than 0.5 g/100 mL. The "saccharide" is a type of carbohydrate, one of the three major nutrients, and is a generic name for available carbohydrates calculated by subtracting dietary fiber content from total carbohydrates.

The beverage of the present invention is preferably colorless and/or clear. In general, beverages and foods with a colorless clear appearance like water are likely to be perceived to taste salty, and this tendency is particularly strong in beverages. According to this invention, a beverage that has an excellent flavor balance and retains a refreshing sensation can be obtained. Further, the beverage of this invention can be perceived as having an excellent taste even when it is drunk directly from a package with a small opening, such as PET bottle.

By stating that "the beverage is clear", it is meant that the beverage is not white turbid like so-called sports drinks nor cloudy like cloudy fruit juices, and is visually clear like water. The degree of clearness of the beverage can be converted to numerical data using, for example, a known technique for measuring liquid turbidity. For example, beverages whose absorbance at a wavelength of 660 nm as measured using an ultraviolet visible spectrophotometer (*e.g.,* UV-1600 (produced by Shimadzu Corporation)) is not more than 0.06 can be regarded as "clear". The beverage of this invention preferably has an absorbance at a wavelength of 660 nm of not more than 0.02, more preferably not more than 0.01.

By stating that "the beverage is colorless", it is meant that the beverage does not have a visually perceivable color. The color of the beverage can be converted to numerical data using, for example, a known technique for measuring object's color difference. For example, beverages whose ΔE value of transmitted light as measured with respect to pure water using a colorimetric color difference meter (ZE 2000 (produced by Nippon Denshoku Industries Co., Ltd.)) is not more than 3.5 can be regarded as "colorless".

In a preferred mode, the pH of the beverage of the present invention is preferably adjusted to acidic pH, from the viewpoints of long-term storage and microbial contamination. To be specific, the pH of the inventive beverage is preferably adjusted to be in the range of 2 to 5, more preferably 2.5 to 4.5, still more preferably 3 to 4. In this connection, since the pericarp extract of this invention can be produced without using fruit juice, there occurs no decrease in pH due to organic acids derived from fruit juice. Thus, even when the inventive pericarp extract is added in large amounts to a beverage with around neutral pH (pH 5 to 9), which is other than a fruit juice beverage, the pericarp extract is unlikely to affect the pH of the beverage. For this reason, it is also desirable to use the pericarp extract in a beverage with a pH of 5 to 9.

The acidity of the beverage of the present invention is preferably adjusted to be in the range of 0.05 to 0.3 g/100 mL, more preferably about 0.06 to 0.25 g/100 mL, still more preferably about 0.07 to 0.2 g/100 mL, in terms of citric acid. By adjusting acidity to lie in such a range, a particularly easy-to-drink beverage with a moderately acidic taste can be obtained. The acidity of a beverage can be measured by a common titration method.

Examples of acidulants include, but are not limited to, different acids such as citric acid, succinic acid, lactic acid, malic acid, tartaric acid, gluconic acid, and phosphoric acid, or salts thereof. The amount of acidulant used is not uniquely determined and varies with the type of acidulant used and the like, but is typically in the range of 0.01 to 5 mass%, preferably 0.05 to 0.5 mass%, with respect to the amount of the beverage.

The beverage of the present invention may be provided as a carbonated beverage containing carbon dioxide gas. Addition of carbon dioxide gas can be performed using a method commonly known to skilled artisans. In order to achieve this purpose: for example, without limitation, carbonic water containing carbon dioxide may be added; carbon dioxide may be dissolved under pressure in a beverage; carbon dioxide and a beverage may be mixed in piping using a mixer such as a carbonator produced by Tuchenhagen GmbH; a beverage may be sprayed into a tank filled with carbon dioxide to cause the beverage to absorb carbon dioxide; or a beverage may be mixed with carbonic water to produce a carbon dioxide gas-containing beverage.

In one mode, the beverage of the present invention can be made into a carbonated beverage. In this process, the pressure of carbon dioxide gas is preferably adjusted to such a level that the carbonated beverage can be perceived as having a refreshing sensation coming from carbon dioxide gas, and is preferably in the range of 0.5 to 4.5 kgf/cm², more preferably 1.0 to 3.0 kgf/cm², still more preferably 1.5 to 2.5 kgf/cm², as measured by the carbon dioxide gas pressure measurement method described below.

### Beverage production method

One aspect of the present invention can be understood to relate to a method for producing a packaged beverage. Since the beverage of this invention comprises a pericarp extract, the beverage production method according to this invention involves the step of adding a pericarp extract. For the purpose of producing a packaged beverage, the method involves at least the steps of preparing a beverage having a pericarp extract added thereto, and packing the prepared beverage in a package.

The beverage of the present invention can be produced using a conventionally known method. Any skilled artisan can design, as appropriate, the conditions for an extract addition step, an optional sterilization step, and a packaging step.

Another aspect of the present invention can be understood to relate to a method for enhancing the taste of a beverage through addition of a pericarp extract.

### EXAMPLES

Hereunder, the present invention will be described in more detail by way of specific experimental examples, but this invention is not limited to these experimental examples. Unless otherwise stated herein, all concentrations and other like parameters are expressed on a mass basis, and all numerical ranges are inclusive of their endpoints.

### Experiment 1: Amino acid measurement in pericarp hydrolysates

The four different citrus pericarps indicated below were analyzed for amino acid profile. The amino acid profiles of these citrus pericarps were quantified according to the analytical method specified in "Standards Tables of Food Composition in Japan - 2015 - (Seventh Revised Edition), Amino acid Composition Tables". To be specific, the citrus pericarps were hydrolyzed with performic acid and hydrochloric acid (for methionine measurement), barium hydroxide (for tryptophan measurement), or hydrochloric acid (for measurement of other amino acids), and then the resulting hydrolysates were quantified for amino acid content.
- Lemon pericarp
- Grapefruit pericarp
- Pink grapefruit pericarp
- Orange pericarp

The quantification results are shown in the table given below. It should be noted that, since the samples analyzed were hydrolyzed ones, the glutamic acid content includes glutamine, the aspartic acid content includes asparagine, and the cystine content represents a cysteine content.

**[Table 1]**

| | | Amino acid concentration in pericarp hydrolysate supernatant (ppm) | | | |
|---|---|---|---|---|---|
| | | Lemon | Grapefruit | Pink grapefruit | Orange |
| Amino acid | Arginine | 460 | 440 | 310 | 850 |
| | Lysine | 440 | 490 | 410 | 700 |
| | Histidine | 190 | 220 | 170 | 310 |
| | Phenylalanine | 300 | 400 | 330 | 440 |
| | Tyrosine | 230 | 290 | 250 | 430 |
| | Leucine | 450 | 530 | 470 | 680 |
| | Isoleucine | 250 | 310 | 270 | 390 |
| | Methionine | 80 | 100 | 80 | 150 |
| | Valine | 330 | 390 | 340 | 520 |
| | Alanine | 370 | 450 | 380 | 610 |
| | Glycine | 340 | 460 | 390 | 560 |
| | Proline | 690 | 910 | 570 | 2010 |
| | Glutamic acid | 740 | 910 | 630 | 1160 |
| | Serine | 410 | 410 | 320 | 600 |
| | Threonine | 270 | 330 | 270 | 450 |
| | Aspartic acid | 2440 | 2300 | 720 | 5040 |
| | Tryptophan | 100 | 160 | 140 | 210 |
| | Cystine | 120 | 180 | 130 | 200 |
| Total | | 8090 | 9100 | 6050 | 15110 |

### Experiment 2: Enzymatic treatment of a pericarp slurry

### (2-1) Enzymatic treatment with different enzymes

Lemon pericarp was crushed into a size of 2 mm using a high-speed grinder (MICRO MEISTER 3M7-40, produced by Masuko Sangyo Co., Ltd.), and then water was added to prepare a slurry (concentration: 22%). Next, each of different enzymes was added to enzymatically treat the slurry under stirring at 40°C for 2 hours. Thereafter, the reaction solution was heated over hot water bath to induce enzyme deactivation, and was centrifuged to separate into solid and liquid phases, whereby a pericarp extract was obtained as a supernatant. In this experiment, each of the following enzyme preparations was added to give a concentration of 4 w/w% per slurry. In the case of combined addition of a pectinase and a hemicellulase, each of these enzymes was added at a concentration of 2 w/w%.
- Pectinase (Sucrase N, produced by San-Ei Gen F.F.I., Inc.)
- Cellulase (Cellulase T "Amano" 4, produced by Amano Enzyme Inc.)
- Hemicellulase (Hemicellulase "Amano" 90, produced by Amano Enzyme Inc.)
- Pectinase (Sucrase N, produced by San-Ei Gen F.F.I., Inc.) and hemicellulase (Hemicellulase "Amano" 90, produced by Amano Enzyme Inc.)

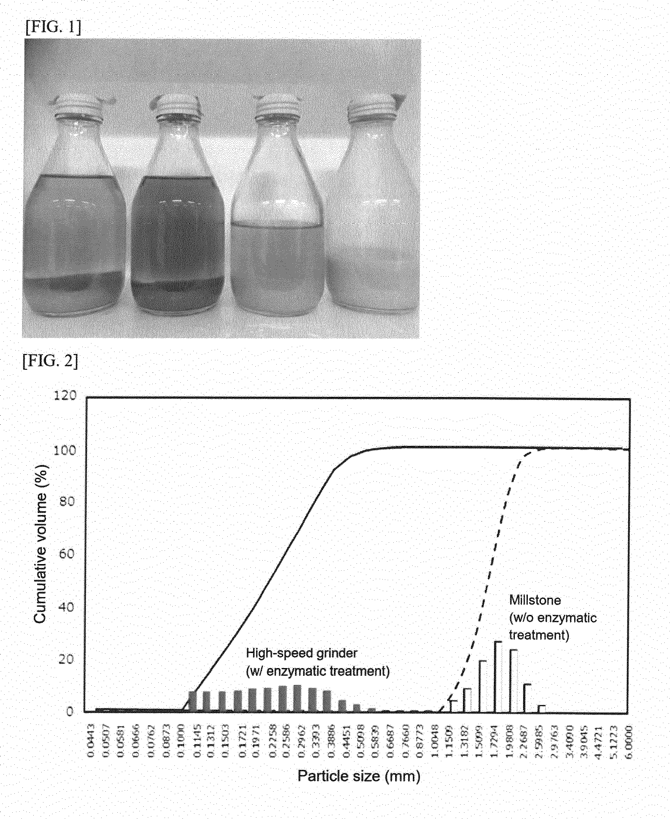

FIG. 1 shows a photograph showing the appearances of slurries settled overnight after enzymatic treatment (starting from the left; hemicellulase + pectinase-treated slurry, hemicellulase alone-treated slurry, pectinase alone-treated slurry, and cellulase alone-treated slurry). The pectinase alone-treated and cellulase alone-treated slurries were observed to get cloudy. On the other hand, the hemicellulase-treated slurries did not get cloudy, and given this fact, it was expected that the solid-liquid separation of the hemicellulase-treated slurries by centrifugation, etc. would be completed in a relatively short time.

The resulting pericarp extracts were analyzed by HPLC for amino acid content under the following conditions.

| | |
|---|---|
| - HPLC system: | Waters amino acid analyzer 2695 |
| - Column: | AccQ-Tagcolumn (3.9 mm × 150 mm) |
| - Column temperature: | 40°C |
| - Mobile phase A: | AccQ-TagA (pH 5.8) |
| - Mobile phase B: | Acetonitrile |
| - Mobile phase C: | Water/methanol = 9/1 |
| - Detection: | EX250nm, EM 395 nm, Gain 100 |
| - Reference standards: | Different amino acids |
| - Injection volume: | 5 µL |
| - Gradient program | |

**[Table 2]**

| Time (min.) | Flow rate (mL/min.) | A (%) | B (%) | C (%) |
|---|---|---|---|---|
| 0 | 1 | 100 | 0 | 0 |
| 1 | 1 | 99 | 1 | 0 |
| 16 | 1 | 97 | 3 | 0 |
| 25 | 1 | 94 | 6 | 0 |
| 35 | 1 | 86 | 14 | 0 |
| 40 | 1 | 86 | 14 | 0 |
| 50 | 1 | 82 | 18 | 0 |
| 51 | 1 | 0 | 60 | 40 |
| 54 | 1 | 100 | 0 | 0 |
| 75 | 1 | 0 | 60 | 40 |
| 110 | 0 | 0 | 60 | 40 |

The results of amino acid content analysis of the resulting pericarp extracts are shown in the table given below. It was revealed that when pericarp slurries were treated with a hemicellulase, the amino acid content was increased to not less than about 1.5-fold as compared to the case of no addition of enzyme. It was also found that when a pericarp slurry was treated with a hemicellulase alone (4% hemicellulase), slightly larger amounts of amino acids were extracted as compared to the case of the treatment with a combination of a hemicellulase and a pectinase (2% hemicellulase + 2% pectinase). In common usage, the flowability of pericarp slurries is sometimes enhanced using a pectinase, but it was found that it is not necessarily required to use a pectinase to increase the amino acid content in pericarp extracts.

**[Table 3]**

| Enzyme used | Amino acid content (ppm) |
|---|---|
| Hemicellulase + pectinase | 3007 |
| Hem icellulase | 3229 |
| Pectinase | 2348 |
| Cellulase | 2361 |
| No enzyme | 2000 |

### (2-2) Enzymatic treatment with a protease and a pectinase

The treatment of a pericarp extract with a protease was also investigated. To be specific, different pericarp extracts were produced by the same procedure as in Experiment 2-1 except that enzymatic treatment was performed as described below.
(Sample 1) 0.1 w/w% pectinase (Sucrase N, produced by San-Ei Gen F.F.I., Inc.), with respect to the weight of pericarps, was added to a pericarp slurry to enzymatically treat the slurry under stirring at 40°C for 2 hours.
(Sample 2) 0.1 w/w% pectinase (Sucrase N, produced by San-Ei Gen F.F.I., Inc.) and 1 w/w% protease (Protease "Amano" A, produced by Amano Enzyme Inc.), with respect to the weight of pericarps, were added to a pericarp slurry to enzymatically treat the slurry under stirring at 40°C for 2 hours.

The resulting pericarp extracts were quantified for amino acid content. As a result, the amino acid concentration in pericarp extract was increased by about 32% in the case of pectinase + protease treatment (Sample 2) as compared to the case of pectinase alone treatment (Sample 1). In other words, in the sample treated with a combination of a pectinase and a protease, the amino acid concentration in pericarp extract was increased to about 1.32-fold, as compared to that of the sample treated with a pectinase alone.

### (2-3) Enzymatic treatment with a protease and a hemicellulase

Enzymatic treatment with a combination of a hemicellulase and a protease was performed. To be specific, different pericarp extracts were produced by the same procedure as in Experiment 2-1 except that enzymatic treatment was performed as described below.
(Sample 3) 1 w/w% hemicellulase (Hemicellulase "Amano" 90, produced by Amano Enzyme Inc.), with respect to the weight of pericarps, was added to a pericarp slurry to enzymatically treat the slurry under stirring at 40°C for 2 hours.
(Sample 4) 1 w/w% hemicellulase and 1 w/w% protease (Orientase AY, produced by HBI Enzymes Inc.), with respect to the weight of pericarps, were added to a pericarp slurry to enzymatically treat the slurry under stirring at 40°C for 2 hours.
(Sample 5) 1 w/w% hemicellulase (Hemicellulase "Amano" 90, produced by Amano Enzyme Inc.), with respect to the weight of pericarps, was added to a pericarp slurry to enzymatically treat the slurry under stirring at 40°C for 2 hours, and then 1 w/w% protease was further added to enzymatically treat the slurry under stirring at 40°C for 2 hours.

**[Table 4]**

| | Treatment time (min.) and amino acid concentration (ppm) | | | | |
|---|---|---|---|---|---|
| | 0 | 30 | 60 | 90 | 120 |
| Sample 3 (hemicellulase alone) | 2890 | 3210 | 3340 | 3340 | 3550 |
| Sample 4 (hemicellulase + protease) | 2890 | 4380 | 4190 | 4390 | 4450 |
| Sample 5 (hemicellulase followed by protease) | 3550 | 4440 | 4350 | 4760 | 4700 |

The table given above shows the relationship between enzymatic treatment time and amino acid concentration in the resulting pericarp extracts. Additionally, the treatment times for Sample 5 are times of treatment with a protease.

According to comparison between Sample 3 (treated with a hemicellulase alone) and Sample 4 (treated with a combination of a hemicellulase and a protease), it is found that amino acids can be extracted in larger amounts by combined treatment with a hemicellulase and a protease. Further, according to comparison between Sample 4 (simultaneously treated with a hemicellulase and a protease) and Sample 5 (sequentially treated with a hemicellulase followed by a protease), it is found that when enzymatic treatment is performed using a hemicellulase and a protease, simultaneous treatment with these enzymes leads to more significant reduction of the total enzymatic treatment time required to extract a similar amount of amino acids. To put it another way, the amino acid concentration in Sample 5 reached more than 4000 ppm only after completion of enzymatic treatment for a total of 150 minutes, which consisted of the treatment with a hemicellulase for 120 minutes followed by the treatment with a protease for 30 minutes. In contrast, the amino acid concentration in Sample 4 (enzymatically treated simultaneously with a hemicellulase and a protease) reached more than 4400 ppm after completion of enzymatic treatment for a total of 30 minutes.

### Experiment 3: Investigation of production conditions

### (3-1) Investigation of pericarp thickness

Investigation was made of the different thicknesses of pericarps collected from lemon fruits. According to the measurement of fruit juice collected from a lemon fruit sample using a juice extractor, the weight percentages of fruit juice and pericarp were about 65% and about 35%, respectively.

Pericarps with a thickness of 2.5 to 4 mm were collected from lemon fruits using a juice extractor (Polycitrus, produce by Fratelli Indelicate). As shown in the table given below, it was found that when pericarp was collected with a thickness of 4 mm from a lemon fruit, a small quantity of fruit juice got mixed in with the pericarp.

**[Table 5]**

| Pericarp thickness (mm) | Weight percentage of collected pericarp (%) | Result |
|---|---|---|
| 2.5 | 25 | Some more pericarp could be collected. |
| 3.0 | 33 | Enough pericarp was collected with no fruit juice mixed in. |
| 4.0 | 37 | A pericarp extract would taste acidic because fruit juice was mixed in. |

### (3-2) Enzymatic treatment and slurry flowability

A pericarp extract was produced using a hemicellulase (Hemicellulase "Amano" 90, produced by Amano Enzyme Inc.) by the same procedure as in Experiment 2-1.

The resulting pericarp extract was found to be significantly highly flowable. After centrifugation, the slurry was measured for particle size distribution by laser diffraction, and the results showed that the particle size was distributed in the range of 100 to 700 µm, and in particular that 90% (by cumulative volume) of particles were distributed below 500 µm (FIG. 2; x-axis: volume (%), y-axis: particle size (µm)).

For the purpose of reference, another pericarp slurry was produced by the same procedure as in Experiment 2-1, except that a millstone grinder (MKZA10-15JIV, produced by Masuko Sangyo Co., Ltd.) was used instead of a high-speed grinder and that no enzymatic treatment was applied, and the produced slurry was likewise measured for particle size distribution. The results for this slurry are also shown in the same figure. In the slurry obtained through millstone grinding, most particles were distributed in the range of 1 to 3 mm.

### (3-3) Enzymatic treatment by multistep addition of pericarp

Different methods for addition of pericarp as a source material were investigated as described below.

### (A) Enzymatic treatment by single-step addition

A lemon pericarp slurry was prepared by the same procedure as in Experiment 2-1 except that the concentration of the pericarp slurry was adjusted to 70%. Next, a hemicellulase was added to enzymatically treat the slurry under stirring at 40°C for one hour.

### (B) Enzymatic treatment by multistep addition

A lemon pericarp slurry with a concentration of 50% was prepared using five seventh of the same pericarp as used in Experiment A, and a hemicellulase was added to enzymatically treat the slurry under stirring at 40°C for 30 minutes. Next, the remaining two seventh of the pericarp was added, and the slurry was further enzymatically treated under stirring at 40°C for 30 minutes.

### (Results of experiments)

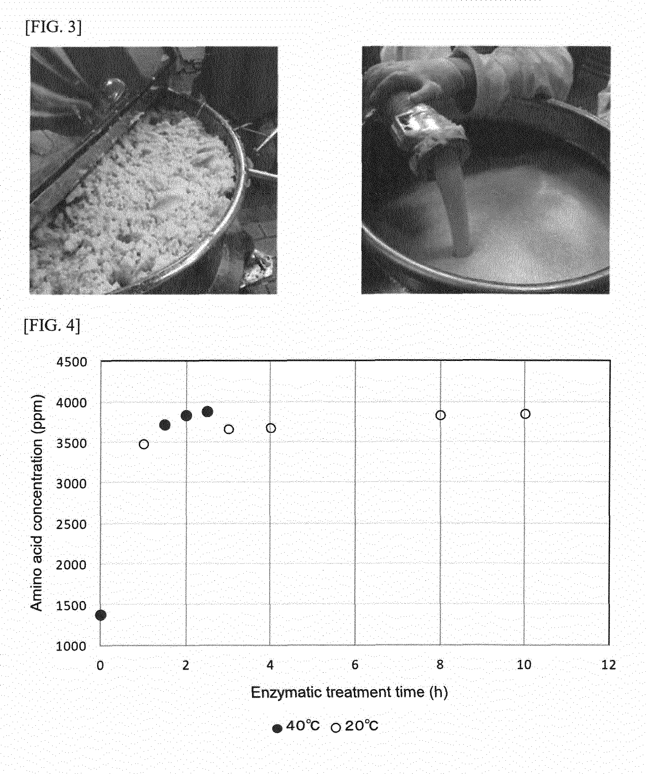

In the case of single-step addition in Experiment A, bridges were formed between pericarp solids in the slurry, and as such, the enzymatic reaction did not proceed well, and the slurry remained less flowable even after completion of the treatment for one hour (left panel in FIG. 3).

On the other hand, in the case of multistep addition in Experiment B, the pericarp slurry was observed to get flowable after 30 minutes, with no bridge formed between pericarp solids, and even after the lapse of one hour, the pericarp slurry remained sufficiently flowable (right panel in FIG. 3).

### (3-4) Investigation of slurry concentrations

Different pericarp extracts were prepared by the same procedure as in Experiment 2-1 except that different concentrations of lemon pericarp slurries were used. However, in this experiment, 1 w/w% hemicellulase (Hemicellulase "Amano" 90, produced by Amano Enzyme Inc.) and 1 w/w% pectinase (Sucrase N, produced by San-Ei Gen F.F.I., Inc.), with respect to the weight of pericarps, were added to the pericarp slurries to effect enzymatic reaction.

The resulting pericarp extracts were measured for amino acid concentration. The measurement results are shown in the table given below. As evident from the table, it was shown that as the concentration of pericarp slurry became higher, the amino acid concentration in pericarp extract got higher. However, it was found that since resistance to stirring increases with increasing concentration of pericarp slurry, slurry concentration should be set depending on the performance of stirrer.

**[Table 6]**

| Slurry concentration (%) | Amino acic concentration (ppm) |
|---|---|
| 22 | 1892 |
| 40 | 2972 |
| 50 | 3594 |
| 60 | 4190 |

### (3-5) Investigation of temperature conditions (protease)

Different pericarp extracts were prepared by the same procedure as in Experiment 2-1 except that 2 w/w% protease (Protease A, produced by Amano Enzyme Inc.) was used with respect to the weight of pericarps. However, in this experiment, the enzymatic reaction took place with the enzymatic treatment temperature being varied from 20 to 60°C.

The resulting pericarp extracts were measured for amino acid concentration. The measurement results are shown in the table given below and FIG. 4. In the case of enzymatic treatment at 40°C (●), increased amino acid concentration in pericarp extract was obtained in a shorter period of time than in the case of enzymatic treatment at 20°C (○).

Further, according to the sensory evaluation performed to determine the flavor of the produced pericarp extracts, the pericarp extract obtained through treatment at 20°C was superior in flavor over the one obtained through treatment at 40°C. From the perspective of actual production, it may well be considered preferable to allow enzymatic reaction to proceed for a certain length of time in order to ensure constant quality control. For example, enzymatic treatment for 12 hours can be completed by allowing reaction to go overnight. Accordingly, it is considered that even if enzymatic treatment is performed at 20°C, there will be no problem from the viewpoint of process management or cost of production equipment.

**[Table 7]**

| Time (h) | Amino acid concentration (ppm) | |
|---|---|---|
| | 20°C | 40°C |
| 0 | 1381 | 1381 |
| 1.0 | 3481 | - |
| 1.5 | - | 3722 |
| 2.0 | - | 3838 |
| 2.5 | - | 3885 |
| 3.0 | 3664 | - |
| 4.0 | 3681 | - |
| 8.0 | 3835 | - |
| 10.0 | 3854 | - |
| 12.0 | 3940 | - |

### (3-6) Enzyme deactivation

Since crushed citrus pericarps are enzymatically treated, there is a possibility that residual enzyme activity may affect the quality of a pericarp extract. Thus, different enzyme deactivation conditions were investigated as described below.
(A) The same procedure as in Experiment 2-2 (for Sample 2) was followed to prepare a product enzymatically treated with a protease and a pectinase. Then, the resulting product was centrifuged to separate into solid and liquid phases, and the supernatant was heated over hot water bath to deactivate the enzymes, whereby a pericarp extract was obtained. The heating over hot water bath was done at 95°C for 5 minutes.
(B) The same procedure as in Experiment 2-2 (for Sample 2) was followed to prepare a product enzymatically treated with a protease and a pectinase. Then, the resulting product was centrifuged to separate into solid and liquid phases, and the supernatant was passed through a spinning cone column (SCC) to deactivate the enzymes by heating in countercurrent contact flow, whereby a pericarp extract was obtained. The conditions for retention in the SCC were at 100°C for about one minute.

As a result of measuring the amino acid contents in the resulting pericarp extracts, the pericarp extracts obtained in Experiments A and B had an amino acid content of 6136 ppm and 5825 ppm, respectively, which were almost the same. Further, according to the sensory evaluation actually performed on the obtained pericarp extracts, the pericarp extract in Experiment B was perceived to taste slightly bitter, and such a bitter taste was regarded as favorable since it would serve as a characteristic of a beverage.

In the process of preparation of the pericarp extract in Experiment B, enzyme deactivation was achieved by heating in a shorter period of time; therefore, it is considered that there is no temporal variation in amino acid concentration, and that deterioration of pericarp extract quality caused by thermal history is suppressed. Further, since the SCC was used for treatment, the process in Experiment B was considered to enable simultaneous collection of not only a pericarp extract but also a suitable aroma component.

### (3-7) Organic acid concentration measurement

Since the pericarp extract of the present invention has no fruit juice mixed therein, amino acid concentration alone can be enhanced by adding the inventive pericarp extract to a beverage. In this experiment, commercially available beverages, lemon juice products (produced by Gan-Shmuel Foods Ltd.), and the pericarp extract of this invention (*i.e.,* lemon pericarp extract obtained in Experiment 3-5) were measured for amino acid and organic acid concentrations.

Amino acid concentration was quantified by the procedure described in Experiment 2-1. With regard to organic acid concentration, the content of citric acid and malic acid was quantified using a high performance liquid chromatography system (Shimadzu's LC-10 series) under the conditions detailed below.
- Equipment used: Controller (SCL-10A), delivery pump (LC-10AD), degasser (DGU-14A), column oven (CTO-IOAC), autosampler (SIL-10AD), electroconductivity detector (CDD-6A), chromatopac integrator (C-R7Aplus), columns (Shim-pack SCR-102H (8 mm I.D × 300 mm L); 2 columns in series), and guard column (SCR-102H, 6 mm I.D × 50 mm L)
- Mobile solution: 4.8 g of p-toluenesulfonic acid was pipetted out in a 1 L volumetric flask and made up to the mark with distilled water. Upon use, the solution was diluted 5-fold, passed through a 0.45 µm membrane filter, and the filtrate was used as a mobile solution.
- Reaction solution: To prepare a solution to be reacted after passing through columns, 4.8 g of p-toluenesulfonic acid, 150 mg of EDTA and 20.9 g of Bis-Tris were pipetted out in a 1 L volumetric flask and made up to the mark with distilled water. Upon use, the solution was diluted 5-fold, passed through a 0.45 µm membrane filter, and the filtrate was used as a reaction solution.

| | |
|---|---|
| - Flow rate: | 1.6 mL/min. |
| - Detection: | Electroconductivity detection (polarity: +) |
| - Sample injection volume: | 10 µL |
| - Analysis time: | 30 min. |

**[Table 8]**

| Sample | Amino acid (ppm) | Total concentration of citric acid and malic acid (ppm) | (Citric acid+malic acid)/ amino acids (by weight) |
|---|---|---|---|
| Lemon pericarp extract (enzymatically treated at 20°C in Experiment 3-5; Brix 13) | 3440 | 6340 | 1.8 |
| Clear lemon juice concentrate (7x concentrate, acidity 31.5, Brix 42) | 18700 | 411000 | 22.5 |
| Lemon juice 1 (clear, acidity 37, Brix 24) | 11700 | 327000 | 28.1 |
| Lemon juice 2 (clear, acidity 42, Brix 31.5) | 14000 | 430000 | 30.8 |
| Lemon juice 3 (cloudy, acidity 40, Brix 30) | 10800 | 441000 | 40.9 |
| Lemon-flavored near-water | 1.5 | 1520 | 1013.3 |
| Lemon-flavored water (with 1% lemon juice) | 30.5 | 3790 | 124.3 |
| Lemon juice (100% juice from concentrate) | 2910 | 62000 | 21.3 |
| Lemon tea | 43 | 997 | 23.2 |
| Carbonated beverage with lemon, orange & grapefruit juices (with <10% juice) | 152 | 6550 | 43.1 |
| Carbonated beverage with lemon juice (with 1% juice) | 22.3 | 4440 | 199.1 |
| Lemon-flavored, non-sugar carbonic water | 19 | 272 | 14.3 |
| Shochu-based beverage with lemon (Company A) | 63.5 | 4560 | 71.8 |
| Shochu-based beverage with lemon (Company B) | 95.1 | 2310 | 24.3 |
| Shochu-based beverage with lemon (Company C) | 51.8 | 5070 | 97.9 |
| Sports drink with lemon peel extract (non-juice) | 0 | 4920 | - |
| Carbonated beverage with orange juice (with 12% juice) | 439 | 3240 | 7.4 |
| Orange juice beverage (100% juice from concentrate) (Company a) | 4460 | 12800 | 2.9 |
| Orange juice beverage (100% juice from concentrate) (Company b) | 2590 | 10900 | 4.2 |
| Orange juice beverage (100% juice from concentrate) (Company c) | 3280 | 10400 | 3.2 |
| Orange juice beverage (100% juice from concentrate) (Company d) | 2940 | 10400 | 3.5 |
| Grapefruit juice beverage (100% juice from concentrate) | 2420 | 11600 | 4.8 |
| Grapefruit juice beverage (100% juice from concentrate) | 2040 | 13300 | 6.5 |

It was found that the lemon pericarp extract of the present invention was lower in weight ratio of (citric acid and malic acid)/amino acids than commercial lemon juice products. All the commercial beverages made with lemon had a weight ratio of (citric acid and malic acid)/amino acids of not less than 14. Therefore, it was considered that with the use of the lemon pericarp extract of this invention, a profound taste can be added to beverages and foods without making them taste intensely acidic, and a fruit juice sensation can be added even to non-juice or low-juice beverages.

### Experiment 4 Production of packaged beverages

### (4-1) Production of packaged beverages 1

Different packaged carbonated beverages were produced by adding the pericarp extract obtained at a reaction temperature of 20°C in Experiment 3-5 (Brix 12.6, amino acid content: 3200 ppm) to commercially available lemon-flavored carbonic water (non-sugar; produced by Suntory Beverage & Food Limited; amino acid content: 19 ppm).

The produced beverages were subjected to sensory evaluation and rated for their profound taste on a 4-point scale according to the following criteria.
4 points: Strongly perceived as having a profound taste.
3 points: Perceived as having a profound taste.
2 points: Slightly perceived as having a profound taste.
1 point: Not perceived as having a profound taste.

**[Table 9]**

| Beverage sample No. (carbonated beverage) | 1 | 2 | 3 |
|---|---|---|---|
| Amino acid concentration in beverage (Concentration of amino acids from pericarp extract) | 19.0 ppm (0 ppm) | 23.3 ppm (4.3 ppm) | 24.4 ppm (5.4 ppm) |
| Sensory rating (profound taste) | 1 | 2 | 3 |

The evaluation results are shown in the table given above. The beverage produced with no addition of pericarp extract (Sample 1) was not perceived as having a profound taste, whereas those produced with addition of the pericarp extract of the present invention were enhanced in taste. In particular, the sample produced by adding the inventive pericarp extract so as to increase the amino acid concentration by 5.4 ppm was a non-sugar carbonated beverage, but was perceived as having a profound taste comparable to a beverage with 1% lemon juice (in terms of straight fruit juice).

### (4-2) Production of packaged beverages 2

Different packaged beverages with 1% or 3% lemon juice in terms of straight fruit juice were produced by adding a commercially available clear lemon juice product (7-fold concentrate, acidity 31.5, Brix 42, produced by Gan-Shmuel Foods Ltd.) to water. Granulated sugar and fructose were added to adjust the Brix values of the beverages to 4.5 (weight ratio of granulated sugar/fructose = 1:1), and citric anhydride and trisodium citrate were added to adjust the pH of the beverages to 3.5.

Separately, different packaged beverages containing a pericarp extract were produced without using lemon juice, by adding the pericarp extract obtained in Experiment 3-5 (Brix 12.6, amino acid content: 3200 ppm) to water. Granulated sugar and fructose were added to adjust the Brix values of the beverages to 4.5 (weight ratio of granulated sugar/fructose = 1:1), and citric anhydride and trisodium citrate were added to adjust the pH of the beverages to 3.5.

**[Table 10]**

| Beverage sample | Lemon pericarp extract-containing beverage | | | | | Lemon juice-containing beverage | |
|---|---|---|---|---|---|---|---|
| | | | | | | 1% lemon juice | 3% lemon |
| Amino acid concentration in beverage (ppm) | - | 5.4 | 10.8 | 16.2 | 21.6 | 27.0 | 81.0 |
| Sensory rating (profound taste) | 1 | 3 | 3 | 4 | 4 | 4 | 4 |

The produced beverages were subjected to sensory evaluation for profound taste by the same procedure as in Experiment 4-1. The results of the sensory evaluation are shown in the table given above. By means of addition of the pericarp extract of the present invention, a profound taste comparable to that of lemon juice-containing beverages was added to beverages. As compared to the beverage containing 1% lemon juice, the samples produced with addition of the inventive pericarp extract were perceived as having a natural lemon sensation. In contrast, the beverage containing 3% lemon juice was not only perceived as having a profound taste, but also perceived as tasting intensely acidic, as deteriorating in flavor, and as having deterioration odor.

## Claims

1. A method for producing a pericarp extract, the method comprising the steps of:
subjecting citrus pericarp made into a size of not more than 5 cm to enzymatic treatment with a hemicellulase and/or a protease to give an enzymatically treated slurry; and
subjecting the enzymatically treated slurry to solid-liquid separation to give a pericarp extract.

2. The method according to claim 1, wherein the solid-liquid separation is performed using a filter press and/or centrifugation.

3. The method according to claim 1 or 2, wherein the citrus pericarp comprises any one or two or more of lemon pericarp, grapefruit pericarp, pink grapefruit pericarp, and orange pericarp.

4. The method according to any of claims 1 to 3, wherein the citrus pericarp comprises lemon pericarp.

5. The method according to any of claims 1 to 4, wherein 90% of particles present in the enzymatically treated pericarp slurry have a particle size of not more than 500 µm.

6. A pericarp extract produced by the method according to any of claims 1 to. 5

7. The pericarp extract according to claim 6, wherein the pericarp extract has a weight ratio of (citric acid and malic acid)/amino acids of not more than 10.

8. A method for producing a beverage or food, the method comprising adding a pericarp extract produced by the method according to any of claims 1 to 5 to a beverage or food.

9. The method according to claim 8, wherein the pericarp extract is added to the beverage so as to give a weight ratio of (citric acid and malic acid)/amino acids of not more than 14.

10. A method for enhancing the taste of a beverage or food, the method comprising:
producing a pericarp extract by the method according to any of claims 1 to 5, and adding the pericarp extract to the beverage or food.
